# EUROPEAN PATENT SPECIFICATION

(11) **EP 0 874 630 B1**
(45) Date of publication and mention of the grant of the patent: **20.08.2003**
(21) Application number: 97900205.2
(22) Date of filing: 10.01.1997
(51) Int. Cl.: A61K 31/495, A61K 31/70, A61K 31/47, A61P 35/00

(54) **TOPOISOMERASE II POISON AND BIS-DIOXYPIPERAZINE DERIVATIVE COMBINATION THERAPY**
KOMBINATIONSTHERAPIE VON TOPOISOMERASE II TOXINEN UND BIS-DIOXYPIPERAZINDERIVATEN
POISON TOPO-ISOMERASE II ET THERAPIE ASSOCIEE A BASE DE DERIVES DE BIS-DIOXYPIPERAZINE

(30) Priority: 11.01.1996 DK 2296; 20.02.1996 US 603105
(43) Date of publication of application: 04.11.1998
(73) Proprietor: TopoTarget ApS, 2100 Copenhagen O (DK)
(72) Inventor: Lensen, Peter Buhl, 3520 Farum (DE); Sehested, Maxwell, 2100 Copenhagen O (DK)
(74) Representative: Plougmann & Vingtoft A/S
(86) International application number: DK9700013
(87) International publication number: WO97025044

(56) References cited:
- EP-A- 0 393 575
- WO-A-86/00812
- WO-A-92/08460
- GB-A- 2 257 430
- US-A- 5 242 901
- US-A- 5 587 382
- BIOCHEMICAL PHARMACOLOGY, vol. 46, 1993, pages 389-393, XP000646923 M. SEHESTED, P.B. JENSEN ET AL: "Antagonistic effect of the cardioprotector (+)-1,2-bis(3,5-dioxopiperazinyl-1-yl)prop ane (ICRF187) on DNA breaks and cytotoxicity induced by the topoisomerase II directed drugs daunorubicin and etoposide (VP-16)" cited in the application
- CANCER RESEARCH, vol. 54, 1994, pages 2959-2963, XP000647964 P.B. JENSEN ET AL: "TARGETING THE CYTOTOXICITY OF TOPOISOMERASE II-DIRECTED EPIPODOPHYLLOTOXINS TO TUMOR CELLS IN ACIDIC ENVIRONMENTS" cited in the application
- CANCER RESEARCH, vol. 51, 1991, pages 4903-4908, XP000647075 TANABE ET AL: "INHIBITION OF TOPOISOMERASE II BY ANTITUMOR AGENTS BIS(2,6-DIOXOPIPERAZINE) DERIVATIVES" cited in the application
- ADVANCES IN PHARMACOLOGY AND CHEMOTHERAPY, vol. 19, 1982, pages 249-290, XP000646919 HERMAN ET AL: "BIOLOGICAL PROPERTIES OF ICRF-159 AND RELATED BIS(DIOXOPIPERAZINE) COMPOUNDS " cited in the application

## Description

The present invention relates to the treatment of malignant conditions which are sensitive to topoisomerase II poisons. In particular, the invention relates to a medicament for selectively killing tumour or metastatic cells within the central nervous system of a large mammal such as a human.

### BACKGROUND OF THE INVENTION

Studies concerning patients suffering from primary cancer outside the central nervous system (CNS) show that 20-25% of the patients develop metastasis of the CNS. However, the risk of developing metastases is dependent on the specific cancer form.

The metastatic complications are very often the patient's first symptom of cancer and may give rise to serious neurological complications at a time when the patient is unaffected by the primary cancer.

The treatment of primary cancer and metastasis of the CNS is far from satisfactory. Most measures, whether radiotherapy or chemotherapy, are limited by the fact that the treatment also affects the normal tissue; accordingly, the tolerance of the normal tissue to the specific treatment is of great importance. The most serious side-effect of chemotherapy is myelosuppression.

The target site for cytotoxic drugs differs significantly among cancer drugs. The essential nuclear enzyme topoisomerase II allows the separation of intertwined DNA strands by creating a transient double stranded break in the DNA backbone. This catalytic cycle of topoisomerase II is believed to be the target of some of the most successful antitumour agents used today, e.g. etoposide (VP-16) in the treatment of testicular and small cell lung cancer (2). There is solid evidence that etoposide, as well as a number of other clinically successful antitumour agents such as daunorubicin and doxorubicin (Adriamycin) (28), are active by inhibiting the resealing of the DNA breaks created by topoisomerase II (19, 21). Although the precise cell killing mechanism is unknown, an obligatory step for the cytotoxicity of the topoisomerase II targeting agents is an increase in cleavable complexes between DNA and topoisomerase II (19). This complex mechanism of cell killing is susceptible to drug modulation.

The clinically active DNA intercalating drug, aclarubicin, completely antagonizes the cytotoxicity of topoisomerase II targeting agents such as etoposide, teniposide, m-AMSA, daunorubicin, and oxaunomycin (15, 16, 17). Not only aclarubicin, but also several other DNA binding agents such as ethidium bromide (24), 9-aminoacridines (7, 20), and chloroquine (18) can antagonize the cytotoxicity of topoisomerase II targeting agents. It is believed that these DNA binding drugs inhibit the initial DNA binding step of the enzyme and thereby suppress the interaction between the enzyme, the topoisomerase II targeting drug, and DNA. Recently, more specific interactions with topoisomerase II have been described. Thus, it appears that cation chelating bis-dioxypiperazines may lock topoisomerase II at its magnesium/ATP binding site at the stage of the catalytic cycle where the homodimeric enzyme is thought to be in the form of a closed bracelet surrounding the DNA (22, 23). By locking the enzyme, the bis-dioxypiperazine seems to hinder topoisomerase II poisons from exerting their cytotoxicity. Thus, the bis-dioxypiperazine derivative ICRF-187 abolishes both DNA breaks and cytotoxicity caused by the topoisomerase II poisons etoposide and daunorubicin (25).

The antagonistic effect of ICRF-187 has been observed on the drug daunorubicin (14), but not on the chemically very similar doxorubicin, and this latter topoisomerase II poison is therefore excluded from the invention. Similarly no antagonism on clerocidin has been found.

### BRIEF DISCLOSURE OF THE INVENTION

The present invention provides an effective medicament for the treatment of cancer by utilizing the antagonistic effect of a bis-dioxypiperazine on a topoisomerase II poison.

More specifically, the present invention relates to a medicament for the treatment of malignant conditions which are sensitive to topoisomerase II poisons wherein the normal tissue is substantially protected from the poison by the bis-dioxypiperazine whereby the malignant conditions can be treated with higher dosages of the topoisomerase II poison.

The medicament is for administration, e.g to a human, of an effective CNS-tumour- or metastasis-killing amount of a topoisomerase II poison except doxorubicin together with administration of a bis-dioxypiperazine compound. The non-CNS tissue of the patient is preferentially protected against the toxic action of the topoisomerase II poison by the bis-dioxypiperazine compound whereby increased dosages of the topoisomerase II poison are tolerated compared to the conventional administration of the topoisomerase II poison alone.

Topoisomerase II poisons such as etoposide are usually already used in maximally tolerated doses in the clinic, and therefore, dose increments which otherwise might have overcome drug resistance are not feasible. However, use of an antagonist together with an agonist according to the present invention may give new prospects. However, if such models of manipulation of etoposide effect are to be useful; they should enable significant dose escalations *in vivo*.

Here it is demonstrated that ICRF-187 in a mouse model markedly antagonizes the toxicity of etoposide *in vivo* and accordingly carries the prospect of powerful effect regulation.

Furthermore, it has surprisingly been found that when ICRF-187 is used with etoposide *in vivo*, the normal treatment schedule for etoposide relating to treatment every day in 3 days or in 5 days is not effective. Thus, the simultaneous use of ICRF-187 in the treatment with etoposide involves a completely new approach to the treatment schedule of topoisomerase II poisons.

Etoposide, VP-16, is a well known drug for use in the treatment of several cancers such as breast cancers, testicular cancers, small cell lung cancers, malignant lymphomas, and leukemias. These cancer forms are very often complicated with metastases to the CNS.

According to the present invention, topoisomerase II poisons are drugs such as the above-mentioned etoposide (VP-16), etoposide-phosphate, teniposide (VM-26) (has a potency which is about 10 fold more potent than VP-16), m-AMSA, daunorubicin, and mitoxantrone. Moreover, any topoisomerase II poison which inhibits the relegation step of the nuclear enzyme topoisomerase II at a step where the enzyme has created a cleavable complex in DNA is within the scope of the invention.

In the present context the term "conditions which are sensitive to topoisomerase II poisons" also includes any disease which would normally not be considered "sensitive" due to toxic side effects of the topoisomerase II poisons.

With respect to toxic side effects of the topoisomerase II poisons, the side effect weight loss has also been reduced by simultaneous treatment with ICRF-187. In this respect, the site of protection *in vivo* has been investigated in mice, and by organ histochemistry there appeared to be less toxicity in the gut by the method according to the present invention compared to treatment with the topoisomerase II poison alone. On the other hand, protection was surprisingly not observed with respect to leucocyte counts as leucopenia was observed in both cases.

According to the present invention, any ratio of the topoisomerase II poison and the bis-dioxypiperazine combination wherein an increased effect is observed compared to the administration of the topoisomerase alone may be used.

In practice, the side effect of VP-16 will be the limiting factor for the maximum dosage to be administered. It is contemplated that the effect of ICRF-187 shows a maximum in a range wherein the drug is relatively non-toxic and therefore VP-16 (etoposide) may be used in relatively high dosages compared to ICRF-187.

With respect to ICRF-187 it will be the side effect of the ICRF-187 which will determine the maximum dosage of this drug. However, it should be noted that in cancer treatment regimes, the cytotoxic treatment is often followed or accompanied by a second therapy for stimulation of the hemopoietic tissue, as myelosuppression such as leucopenia and thrombocytopenia is the side effect which normally limits the dosage of the cytostatic drugs which the patient can tolerate. Accordingly, very high dosages of topoisomerase II poison may be used according to the invention as in such instances the treatment may be performed together with a supporting therapy. In such high dosage treatment schedules, VP-16 may be used in dosages up to 10 g/m².

In the present context, equitoxic and equipotent dosages of topoisomerase II poisons are defined as dosages capable of inducing similar toxic effect or anticancer effect, respectively. By dosage is meant the total amount of drug which is administered in a single administration. Generally, cytotoxic drugs are administered as one daily dosage.

According to the present invention, the term large mammal relates in particular to humans. However, domestic animals, such as dogs, may be treated according to the present invention, if desired. Rodents are not within the scope of a large mammal.

In another aspect, the invention relates to a pharmaceutical kit for selectively killing tumour or metastatic cells within the central nervous system of a large mammal, in particular a human, comprising a dosage unit of a bis-dioxypiperazine and a pharmaceutically acceptable carrier and a dosage unit of topoisomerase II poisons except doxorubicin and a pharmaceutically acceptable carrier. The kit may further comprise the two dosage units in a single infusion system wherein the two dosage units are separated in two individual bags.

The dosage units may comprise the active ingredients as a dry substance, in concentrates suitable for dilution in accordance with the conventional formulation of the drugs, including tablet forms for the topoisomerase II poison.

In one embodiment, the kit comprises the two dosage units in separate containers, e.g. infusion bags which may be administered separately to the patient. In another embodiment, the two containers are connected, e.g by a Y-shaped tube, to a single infusion tube. Furthermore, additional containers, e.g. comprising neutral fluids, may be connected to the kit whereby the infusion tube may be flushed between the separate infusion of each of the drugs.

As mentioned above, etoposide is often used in a 3- to 5-day schedule in patients. However, when etoposide was used in a 5-day schedule in mice, the ICRF-187 mediated protection *in vivo* was not obtained. Protection was observed when etoposide was used once, once every five days, and once every week.

Comparison of different treatment schedules is shown in Table A from which it is clearly apparent that repeated treatments with the combination of the topoisomerase II poison and the bis-dioxypiperazine on day 1, day 3, and day 5 (every two days) result in considerable more deaths than the treatment on day 1, day 5 and day 9 (every four days) or on day 1, day 8, and day 15 (every seven days) even though a small tendency of protection by ICRF-187 is seen in the lower dosages of etoposide of 50 and 70 mg/kg.

### Brief discussion of the results

The present study demonstrates that ICRF-187 antagonizes m-AMSA and etoposide mediated lethal toxicity in mice. Thus, ICRF-187 is an antidote which is effective against both intercalating and non-intercalating types of topoisomerase II poisons. This antagonism is marked as ICRF-187 enables a 3.6 fold increase in LD₁₀ of etoposide, i.e. a 3.6 fold increase in the dose which is killing 10% of the mice. Similar data have been obtained with teniposide resulting in a 3.4 fold increase in LD₁₀ of tenoposide. It is also remarkable that this protection was obtained within a large range of non-toxic ICRP-187 doses.

*In vitro* evidence suggests that this protection is related to an effect on topoisomerase II (22, 25, 27) and not to an inhibition of the formation of toxic free radicals by chelation of free iron. As ICRF-187 is a divalent cation chelator, a hypothesis would be that ICRF-187 interacts at the late magnesium/ATP binding stage of the enzyme's catalytic cycle where the homodimeric topoisomerase II is thought to have the form of a closed bracelet surrounding the DNA. Roca et al. (22) recently performed *in vitro* studies demonstrating a bis-dioxypiperazine induced trapping of the enzyme in the form of a closed protein clamp. By reversible blocking at this stage, ICRP-187 is believed to be able to antagonize the religation inhibition invoked by topoisomerase II poisons.

However, it is not clear whether this marked *in vivo* protection of topoisomerase II poisons by ICRF-187 can be put to clinical use. Accordingly, the antitumour effect of equitoxic doses of etoposide and etoposide plus ICRF-187 was tested in mice bearing L1210 leukemia and a synergistic effect of the combination was found. From the knowledge of antagonism of etoposide cytotoxicity of tumour cells in clonogenic assay (25), no advantage of an i.p./i.p. co-treatment of an ascites tumour with ICRF-187 and etoposide would be expected. In accordance with this, the antitumour effect of equitoxic doses of etoposide and etoposide plus ICRF-187 was almost identical in mice with Ehrlich's ascites tumour cells which are basically confined to the peritoneal cavity (Table 5). In contrast, however, when L1210 cells were used which are known to metastasize readily (26), there was a trend towards a synergistic effect of the drug combination (Table 5). Finally a significant difference between etoposide alone and etoposide and protector was obtained when L1210 cells were inoculated into the cerebrum. Similar data have been obtained with Ehrlich's ascites tumour cells.

Although it is envisaged that an i.v. administration of the topoisomerase II poison and its antagonist ICRF-187 only has a clear effect when the tumour is situated within the CNS, the combination of the two drugs can be beneficial in other settings. Thus, ICRF-187 can protect systemically when the topoisomerase II poison is administered into a tumour containing a confined compartment. Such a compartment could be the peritoneal cavity or the pleural cavity, i.e. the ICRF-187 compound is administered i.v. whereas the topoisomerase II poison is administered locally. This approach can be useful e.g. in the treatment of mesothelioma or metastases from breast of lung cancer in the pleura or treatment of metastases of ovarian cancer and mammary cancer in the peritoneum. The optimum timing of such administrations can vary according to the specific compartment, the specific tumour and the topoisomerase poison.

In the conventional treatment schedules for cancer therapy, dosages to be received are calculated on the basis of the patients' surface area. Generally, the formula of Du Bois is used for calculation of the surface of an individual as follows: A = W^{0.425} x H^{0.725} x 0.007184 wherein A = area (m²), W = weight (kg) and H = height (cm).

The formula has been converted to a normogram wherein the relevant area can be read based on weight and height.

The corresponding dosages for a human and mice can be calculated according to the above, taken together with the conversion factor being so that 1 mg/kg in a large animal, such as a human of a height of 180 cm and weighing 70 kg corresponding to a surface area of about 1.87 m², corresponds to 3 mg/kg in a mouse.

It is believed that when used in the CNS the benefit of the medicament according to the present invention is an interaction of the antagonism of the drugs on the one hand and the pharmacological obstacle of the blood brain barrier on the other hand. Etoposide and its analogue teniposide pass the blood brain barrier (6), and the concentration of these drugs is especially high in CNS-metastases. In contrast, the concentration of the hydrophile ICRF-187 is very low in the cerebrospinal fluid (29); therefore, the combination of ICRF-187 and etoposide is especially of value in patients with etoposide sensitive brain tumours or metastases, as the combination would allow significant etoposide dose escalation by way of protection of normal tissues outside the CNS. In this context it is notable that etoposide is the most important single agent in the treatment of small cell lung cancer (SCLC), a disease which very often relapses in the CNS. This combination of drugs may be valuable in patients with SCLC and brain metastasis.

However, the principle of protection according to the present invention may be utilized in any other compartment of the body where the protecting bis-dioxypiperazine can be separated from a compartment where the tumour cells are present and where the cytotoxic drug is active.

It has been demonstrated that ICRF-187 is a non-toxic powerful protector of m-AMSA, daunorubicin, teniposide and etoposide toxicity *in vivo* allowing significant dose escalations. The etoposide dose increment made possible by treatment with ICRF-187 in non-toxic doses has surprisingly improved the effectiveness of treatment in mice inoculated intracranially with L1210 tumour cells. ICRF-187 therefore shows promise for increased tumour versus host selectivity.

In another aspect of the invention, the method relates to the treatment of a patient suffering from drug resistance towards a topoisomerase poison. Drug resistance is a condition wherein tumour cell killing cannot be obtained with dosages which have previously shown tumour killing effect.

### DETAILED DISCLOSURE OF THE INVENTION

The bis-dioxypiperazine compounds, to which the present invention relates, are bis(3,5-dioxopiperazine-1-yl) alkanes having a structure as shown in the general formula I: 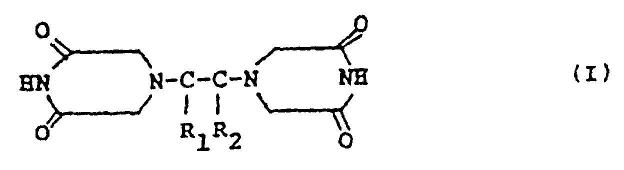 wherein R¹ is not the same as R² and R¹ and R² are hydrogen, or alkyl with 1-4 carbon atoms. The compounds may be in the (-) levo, (+) dextro or (+/-) racemic form. Preferably, R¹ is methyl and R² is hydrogen.

In a first aspect the present invention relates to a medicament for selectively killing tumour or metastatic cells within the central nervous system of a large mammal, in particular a human, which is administered to a mammal an effective CNS-tumour- or metastasis-killing amount of a topoisomerase II poison except doxorubicin, and protecting non-CNS tissue of the mammal against the toxic action of the topoisomerase II poison by administration of a bis-dioxypiperazine compound,
the topoisomerase II poison and the bis-dioxypiperazine compound being selected and administered, with respect to their amounts, expressed in mg per m² of the mammal and with respect to the ratio between their amounts, in such a manner that in mice, the corresponding amounts (the conversion factor being so that 1 mg/kg in a large animal, such as a human of a height of 180 cm and weighing 70 kg corresponding to a surface area of about 1.87 m², corresponds to 3 mg/kg in a mouse) will conform to the following criteria:
i) in mice inoculated into the cerebrum with 15x10⁴ Ehrlich's ascites tumour cells or 10×10⁴ L1210 leukemia cells in a volume of 30 µl of isotonic sodium chloride inoculated on day 0 into the temporal region, treatment i.p. on day 2 with the amount of the topoisomerase II poison and with simultaneous treatment with the amount of the bis-dioxypiperazine compound results in an increased life span of at least 125% compared to the increased life span in mice treated with topoisomerase II poison alone in an equitoxic dose (the equitoxic dose being calculated as the dose which in healthy mice results in a similar lethality as the combination); and
ii) in mice treated with the topoisomerase II poison alone and combined with the bis-dioxypiperazine compound, respectively, the LD₁₀ of the combination is at least 25% higher than the LD₁₀ of the topoisomerase II poison alone.
   In an additional aspect related to the above mentioned criterion i), the treatment with the amount of the bis-dioxypiperazine compound results in an increased life span of at least 150%, such as at least 200%, preferably at least 250%, such as about 300%, compared to the increased life span in mice treated with topoisomerase II poison alone in an equitoxic dose as defined above.
   In a further aspect of the invention related to the above mentioned criterion ii) in mice treated with the topoisomerase II poison alone and combined with the bis-dioxypiperazine compound, respectively, the LD₁₀ of the combination is at least 50% higher, such as at least 100% higher, preferably at least 150% higher, than the LD₁₀ of the topoisomerase II poison alone.
   In a still further aspect of the invention related to the above mentioned criterion ii) in mice treated with the topoisomerase II poison alone and combined with the bis-dioxypiperazine compound, respectively, the LD₁₀ of the combination is at least 200% higher, even more preferred at least 250% higher, preferably at least 300% higher, more preferred at least 350% higher, such as 360% higher, than the LD₁₀ of the topoisomerase II poison alone.
   In addition to criteria i) and ii) mentioned above, the method may further conform to the following criterion:
iii) in mice inoculated i.p. with 15×10⁶ Ehrlich's ascites tumour cells on day 0, treatment i.p. on day 4 with the amount of the topoisomerase II poison and with simultaneous treatment with the amount of the bis-dioxypiperazine compound results in an increased life span of at the most 200%, such as at the most 150%, preferably at the most 125%, compared to the increased life span in mice treated with the topoisomerase II poison alone in an equitoxic dose (the equitoxic dose being defined above).

The interesting issue of criterion iii) above is that the model clearly indicates that the protecting effect of the tissue outside the CNS has a clear effect when the tumour is situated within the CNS. However, as the patient may suffer from a primary cancer outside the CNS, the treatment of the patient may be accomplished by conventional treatment of this cancer, e.g. by topoisomerase II poison in conventional dosages.

Generally, according to the present invention, the topoisomerase II poison is to be administered in a dosage amount equivalent to at least 200 mg/m² of etoposide, such as at least 500 mg/m² of etoposide, preferably at least 1000 mg/m² of etoposide, more preferably at least 1200 mg/m² of etoposide, even more preferred at least 1500 mg/m² of etoposide.

It will be obvious to the skilled person that the dosage may vary among the patients and also in the same patient, as the dosages may be calculated based on the patient's leucocyte count, platelet count and other factors relating to the hemopoietic status of the patient at the time of treatment.

Accordingly, in a further aspect of the invention the topoisomerase II poison is to be administered in a dosage amount equivalent to at least 2000 mg/m² of etoposide, such as at least 2500 mg/m² of etoposide, preferably at least 3000 mg/m² of etoposide, such as 3500 mg/m² of etoposide.

Such high dosages are indicated in patients who tolerate the topoisomerase II poison rather well, or because the bis-dioxypiperazine enables the dosages to be increased due to a superior protection of the combination outside the CNS. Cytotoxic drugs are generally rather well tolerated in the CNS compared to other tissues.

The bis-dioxypiperazine compound is preferably to be administered to the mammal in a dosage amount equivalent to at least 200 mg/m² of ICRF-187 such as at least 500 mg/m² of ICRF-187, preferably at least 1000 mg/m² of ICRF-187, more preferably at least 1200 mg/m² of ICRF-187, even more preferred at least 1500 mg/m² of ICRF-187.

The bis-dioxypiperazine compounds according to the present invention are generally well tolerated by patients. Accordingly, if desired, the dosage may be increased. This will often be preferred in situations where a high dosage of topoisomerase poison II needs to be administered, e.g. in a schedule where single very high dosages are administered with long intervals.

Thus, in a further aspect of the invention the bis-dioxypiperazine compound is to be administered to the mammal in a dosage amount equivalent to at least 2000 mg/m² of ICRF-187, such as at least 2500 mg/m² of ICRF-187, preferably at least 3000 mg/m² of ICRF-187, such as 3500 mg/m² of ICRF-187.

As mentioned above, any ratio between the dosage amount of topoisomerase II poison and the dosage amount of the bis-dioxypiperazine which results in an increased survival compared to the equitoxic dosage of the topoisomerase II poison alone is within the scope of the invention. Nevertheless, it is believed that the best results will be obtained with ratios in the range of 1:6 - 6:1, such as 1:4 - 4:1, preferably in the range of 1:3 - 3:1, calculated on the weight of the active drugs. However, in the case of very potent drugs such as VP-26, the bis-dioxypiperazine may be used in relatively higher dosages than indicated with the above-mentioned ratios.

However, also in the situations where the most optimum ratio of the combination for a patient is not known, the ratio between the dosage amount of topoisomerase II poison and the dosage amount of the bis-dioxypiperazine to be administered is preferably in the range of 1:2 - 2:1, more preferably in the range of 1.5:2 - 2:1.5, the most preferred ratio being 1:1, calculated on the weight of the active drugs, as such ratios are believed to be sufficient in most cases.

In a special embodiment of the invention, the topoisomerase II poison is to be administered in a high dosage amount such as any dosage of between 3 and 10 g/m² or even up to 15 g/m². For many reasons, high dosages may be desirable, and up to the present invention, very high dosages would only be administered together with a supporting treatment such as stimulation of the hemopoietic tissue and/or followed by transplantation of hemopoietic tissue.

In a still further embodiment of the invention, the method may comprise additional therapy for stimulation of the hemopoietic tissue and/or transplantation of hemopoietic tissue.

In another embodiment of the invention, the mammal further receives a topoisomerase II poison alone and/or one or more other anticancer agents. This is especially the case when metastases of the CNS are treated.

The topoisomerase II poison and the bis-dioxypiperazine should be administered substantially simultaneously so that the protection of the tissue outside the CNS is present when the topoisomerase II poison exerts its cytotoxic effect.

Accordingly, in one aspect of the invention the bis-dioxypiperazine is administered prior to the administration of the topoisomerase II.

In a further aspect the bis-dioxypiperazine is to be administered in the period of from 72 hours before the administration of the topoisomerase II poison to 24 hours after, such as from 48 hours before to 12 hours after, more preferred from 24 hours before to 6 hours after, even more preferred from 12 hours before to 3 hours after, still more preferred from 6 hours before to 1 hour after, most preferred from 3 hours before to 1 hour after the administration of the topoisomerase II poison.

As the results indicate, it is believed that it may be an advantage to treat the patients with different intervals between the administration of the dosages than in the conventional schedules wherein the patient is treated every day for 3 or 5 days. Accordingly, in one embodiment the topoisomerase II poison is to be administered in a schedule where an administration is separated from the next administration by about 12 hours, such as by 2, 3, 4, 5, 6, or 7 days, although about 24 hours of separation may also be useful.

In another treatment according to the invention, each administration of the schedule is separated from the next administration by at least 2 days. Each administration may also be separated from the next administration by from about 3 days and up to about 3 weeks or even up to about 4 weeks if desired. One method according to the invention is a method wherein each administration is separated from the next administration by about 7 days.

According to the invention, administration of topoisomerase II poison except doxorubicin can be performed in various manners such as i.p., i.v., or by local application, such as localized injection. Likewise, the administration of a bis-dioxypiperazine can be performed i.p. or i.v.

In one aspect the present invention relates to the use of a dosage unit of topoisomerase II poisons except doxorubicin and a dosage unit of a bis-dioxypiperazine together with a suitable pharmaceutical carrier for the preparation of a medicament for the treatment of malignant tumours of the CNS, including metastasis of the CNS of a mammal such as a human.

The present invention also relates to a pharmaceutical kit for selectively killing tumour or metastatic cells within the central nervous system of a large mammal, in particular a human, said kit comprising
a) a dosage unit of a bis-dioxypiperazine and a pharmaceutically acceptable carrier, and
b) a dosage unit of topoisomerase II poisons except doxorubicin and a pharmaceutically acceptable carrier.

### DESCRIPTION OF THE DRAWING

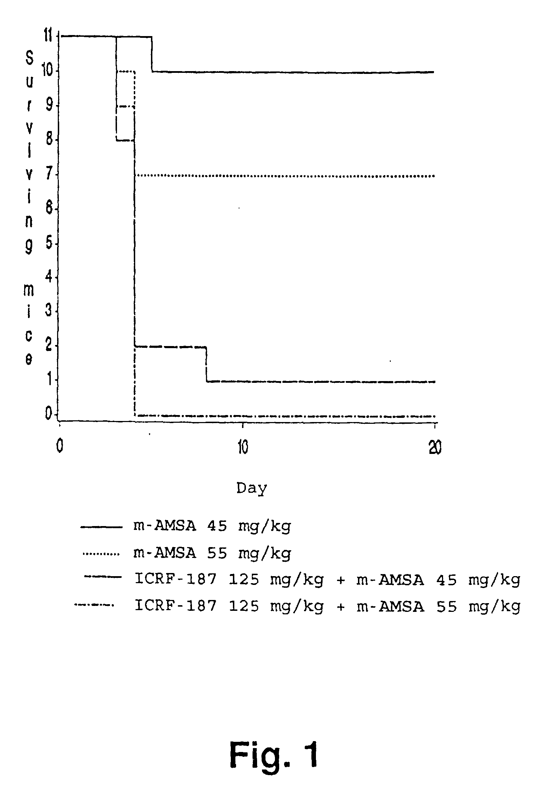

Fig. 1. shows the effect of ICRF-187 on the survival of mice treated with high-dose m-AMSA. Groups of 11 mice were treated as stated in the materials and methods section. Survival is increased in the two groups treated with the combination of drugs as compared to the groups treated with m-AMSA alone. No mice died after day 20.
m-AMSA 45 mg/kg
m-AMSA 55 mg/kg
ICRF-187 125 mg/kg + m-AMSA 45 mg/kg
ICRF-187 125 mg/kg + m-AMSA 55 mg/kg

### MATERIALS AND METHODS

The abbreviations used are: m-AMSA: (4'-(9-acridinylamino)-methanesulfone-m-anisidide, amsakrin). ICRF-187: (ADR-529, dexrazoxane, [(+)-1,2-bis(3,5-dioxopiperazinyl-1-yl)propane]).

ICRF-187 (Cardioxane™) purchased from EuroCetus B.V., Amsterdam, The Netherlands was dissolved in saline to 25 mg/ml. Etoposide (20 mg/ml) and paclitaxel (6 mg/ml) were bought from Bristol-Myers Squibb, Copenhagen, in solution for clinical use. m-AMSA purchased from Parke-Davis, Pontypool, Gwent, United Kingdom, was in N,N-dimetylacetamide solution and further diluted in acid lactose to 5 mg/ml.

### In vivo experiments

First generation hybrids of female random bred Swiss mice and male inbred DBA (NDF1-hybrid) mice were used as previously described (5). The mice weighed between 19 and 21 g at the start of the experiments. Doses of etoposide, m-AMSA, and paclitaxel were adjusted according to weight and ICRF-187 was given in fixed doses either i.p. or i.v. in a tail vein 20 minutes before the investigated drug. Lethality was recorded and surviving mice were sacrificed after 60 days. Toxicity was evaluated by weight and leucocyte counts. For weight evaluations mice were treated with drugs, marked and weighed for eleven consecutive days. Blood for leucocyte counts was obtained from a tail vein and counted in a hemocytometer on days 3, 5 and 7 after treatment.

In therapy studies, mice were inoculated i.p. with either 10⁵ L1210 leukemia cells or 15x10⁶ Ehrlich's ascites tumour cells on day 0 and treated with drugs i.p. on day 4 after inoculation. The brain tumour model consisted of 10⁴ L1210 cells or 15x10⁴ Ehrlich's ascites tumour cells in a volume of 30 µl of isotonic sodium chloride inoculated on day 0 into the temporal region followed by treatment i.p. on day 2, 3 or 4 after inoculation.

Using the log probit method (4, 5, 14) the LD₉₀ (i.e. the dose killing 90% of the animals) of ICRF-187 in mice treated once i.p. was estimated to be 1500 mg/kg and the LD₁₀ to be 500 mg/kg. First the range of ICRF-187 doses capable of protecting against etoposide induced lethality was estimated. In drug combination studies, concentrations in the non-toxic 50 mg/kg to 250 mg/kg range were used except in one experiment, where 500 mg/kg was used. These varying doses were combined with a fixed dose of etoposide of 90 mg/kg. The results are shown in Table 1 and demonstrate that doses of ICRF-187 as low as 50 mg/kg provide good protection.

Next, the dose of etoposide was escalated up to 200 mg/kg and a fixed dose of ICRF-187 was used as protector of etoposide induced lethality. The results are shown in Table 2. ICRF-187 still provides significant protection at 140 mg of etoposide/kg, whereafter the ICRF-187 mediated protection decreases and is negligible at an etoposide dose of 180 mg/kg. LD₁₀ and LD₅₀ values (i.e. doses killing 10% and 50% of the animals, respectively) were computed using the maximum likelihood method on the compiled data (Table 3), showing that while the LD₁₀ of etoposide alone is 34 mg/kg it is 122 mg/kg when combined with 125 mg/kg ICRF-187, corresponding to a dose increment of etoposide of 360%. Similar results were obtained when ICRF-187 was given i.v. and etoposide i.p (data not shown). Thus, ICRF-187 is a powerful protector of etoposide induced lethality in mice. To test whether the ICRF-187 mediated antagonism translated to other topoisomerase II poisons *in vivo*, the toxicity of the combination of ICRF-187 and m-AMSA in healthy mice was investigated using the same schedule as described above. ICRF-187 protects not only against the non-intercalative etoposide, but also against death induced by the DNA intercalative topoisomerase II poison, m-AMSA. As the interaction between ICRF-187 and etoposide is considered to be on the target enzyme topoisomerase II, a non-topoisomerase II directed drug is not expected to be protected by ICRF-187. Therefore, the tubulin-directed drug paclitaxel (Taxol) in combination with ICRF-187 was not expected to be protected. As shown in Table 4 paclitaxel induced lethality cannot be antagonized by ICRF-187.

The effect of combining etoposide and ICRF-187 in mice inoculated i.p. with either Ehrlich's ascites tumour or L1210 tumour cells was then investigated. ICRF-187 had no antitumour effect, while etoposide at 33 mg/kg as expected demonstrated an increase in life span. In combination with ICRF-187 the dose of etoposide was escalated to 120 mg/kg and a non-significant increase in life span in three consecutive experiments using L1210 cells was observed while no synergy was found for Ehrlich's ascites tumour cells (Table 5).

The hypothesis that this difference in increase in life span could be caused by the metastatic potential of the L1210 leukemia cells and a possible difference in transport of the two drugs across the blood brain barrier was investigated. L1210 cells were therefore inoculated into the cerebrum and the animals treated i.p. As demonstrated in Table 6, a significant increase in life span using the high-dose etoposide schedule combined with ICRF-187 was obtained compared to etoposide alone.

The toxicity of drug combinations was evaluated in healthy mice by weight measurements. As shown in Table 7, the etoposide induced weight loss is largely prevented by ICRF-187.

**Table A**

| Comparison of different treatment schedules | | | |
|---|---|---|---|
| Repeated treatments treatment day | 1,3, and 5 (every two days) | 1,5, and 9 (every four days) | 1,8, and 15 (every seven days) |
| etoposide | | | |
| | | | |
| 50 mg/kg | 1/11 | 9/11 | 9/11 |
| 70 - | 0/11 | 2/11 | 0/11 |
| 90 - | 0/11 | 0/11 | 0/11 |
| | | | |

| etoposide + 100 mg/kg ICRF 187 | | | |
|---|---|---|---|
| | | | |
| 50 | 4/11 | 11/11 | 11/11 |
| 70 | 2/11 | 11/11 | 11/11 |
| 90 | 0/11 | 11/11 | 11/11 |
| | | | |
| ICRF-187 | 11/11 | 11/11 | 11/11 |
| 100 mg/kg | | | |
| (survivors/treated) | | | |

**Table 1.**

| Lethality of etoposide alone and combined with ICRF-187. The etoposide dose is fixed and the doses of ICRF-187 vary. | | | | | |
|---|---|---|---|---|---|
| Experiment # | Number of mice | VP-16 mg/kg | Survivors / treated | ICRF-187 + VP-16 mg/kg | Survivors / treated |
| # 1 | 11 | 90 | 1/11 (9%) | 50 + 90 | 11/11 (100%) |
| | | | | 125 + 90 | 11/11 (100%) |
| | | | | 250 + 90 | 11/11 (100%) |
| # 2 | 11 | 90 | 3/11 (27%) | 50 +90 | 9/11 (82%) |
| | | | | 125 + 90 | 11/11 (100%) |
| | | | | 250 + 90 | 11/11 (100%) |
| # 3 | 11 | 90 | 5/11 (45%) | 250 + 90 | 11/11 (100%) |
| | | | | 500 + 90 | 11/11 (100%) |
| # 4 | 11 | 90 | 2/11 (18%) | 50 + 90 | 11/11 (100%)) |
| | | | | 125 + 90 | 11/11 (100%) |
| | | | | 250 + 90 | 11/11 (100%) |

The lethality of etoposide is decreased by pretreatment with ICRF-187 in low, non-toxic doses. Drugs were given once i.p. ICRF-187 was given immediately before etoposide.

**Table 2.**

| Lethality of etoposide alone and combined with ICRF-187. The ICRF-187 dose is fixed and the doses of etoposide vary. | | | | | |
|---|---|---|---|---|---|
| Experiment # | Number of mice | VP-16 mg/kg | Survivors / treated | ICRF-187 + VP-16 mg/kg | Survivors / treated |
| # 1 | 11 | 90 | 8/11 (73%) | 125 + 90 | 11/11 (100%) |
| | | 100 | 2/11 (18%) | 125+100 | 11/11 (100%) |
| | | 120 | 1/11 (9%) | 125+120 | 11/11 (100%) |
| | | 140 | 0/11 (0%) | 125+140 | 11/11 (100%) |
| # 2 | 11 | 90 | 3/11 (27%) | 125 + 90 | 11/11 (100%) |
| | | 100 | 2/11 (18%) | 125+100 | 10/11 (91%) |
| | | 120 | 0/11 (0%) | 125+120 | 8/11 (73%) |
| | | 140 | 1/11 (9%) | 125+140 | 6/11 (55%) |
| # 3 | 11 | 90 | 8/11 (73%) | 125 + 90 | 11/11 (100%) |
| | | 100 | 3/11 (27%) | 125+100 | 11/11 (100%) |
| | | 120 | 1/11 (9%) | 125+120 | 11/11 (100%) |
| | | 140 | 2/11 (18%) | 125+140 | 9/11 (82%) |
| # 4 | 11 | 90 | 3/11 (27%) | 125 + 90 | 9/11 (82%) |
| | | 140 | 1/11 (9%) | 125+140 | 9/11 (82%) |
| | | 180 | 0/11 (0%) | 125+180 | 1/11 (9%) |
| | | 200 | 0/11 0% | 125+200 | 0/11 0% |
| # 5 | 11 | 90 | 3/11 (27%) | 125+90 | 11/11 (100%) |
| | | 140 | 4/11 (36%) | 125+140 | 11/11 (100%) |
| | | 180 | 0/11 (0%) | 125+180 | 5/11 (45%) |
| | | 200 | 2/11 (18%) | 125+200 | 2/11 (18%) |
| # 6 | 11 | 90 | 1/11 (9%) | 125+90 | 11/11 (100%) |
| | | 140 | 0/11 (0%) | 125+140 | 8/11 (73%) |
| | | 180 | 2/11 (18%) | 125+180 | 5/11 (45%) |
| | | 200 | 1/11 1 (9%) | 125+200 | 2/11 (18%) |

In etoposide dose escalation experiments ICRF-187 still provides significant protection at 140 mg of etoposide/kg.

**Table 3.**

| Estimation of LD₁₀ and of LD₅₀ | | | | | | |
|---|---|---|---|---|---|---|
| | log LD₁₀ log LD₁₀ | SE SE | LD₁₀ LD₁₀ mg/kg | log LD₅₀ log LD₅₀ | SE SE | LD₅₀ LD₅₀ mg/kg |
| VP-16 | 1.531 | 0.123 | 34.0 | 1.853 | 0.051 | 71.3 |
| ICRF + VP-16 | 2.088 | 0.009 | 122.4 | 2.214 | 0.011 | 163.8 |

Calculation of LD₁₀ and LD₅₀ using the maximum likelihood estimation. The 6 experiments in Table 2 are included in the computations.

**Table 4.**

| Lethality of paclitaxel alone and combined with ICRF-187. | | | | | |
|---|---|---|---|---|---|
| Paclitaxel mg/kg | Survivors/ treated | % | Paclitaxel + ICRF-187 mg/kg | Survvors/ treated | % |
| 30 | 22/22 | 100 | 125 + 30 | 22/22 | 100 |
| 40 | 12/22 | 55 | 125 + 40 | 11/22 | 50 |
| 50 | 0/22 | 0 | 125 + 50 | 2/22 | 9 |

The compiled data from two experiments showing that paclitaxel induced lethality cannot be protected by pretreamtment with ICRF-187. Groups of 11 mice were treated once i.p. with or without pretreatment with ICRF-187.

**Table 5.**

| Treatment with etoposide and ICRF-187 in tumour bearing mice. | | | | | | | |
|---|---|---|---|---|---|---|---|
| **Ehrlich's tumour cells** | | | | **L1210 tumour cells** | | | |
| Etoposide | | Etoposide + ICRF-187 | | Etoposide | | Etoposide + ICRF-187 | |
| Dose (mg/kg) | | Dose(mg/kg) | | Dose(mg/kg) | | Dose(mg/kg) | |
| 33 | | 120 + 125 | | 33 | | 120 + 125 | |
| N | ILS(%) | ILS(%) | p | N | ILS(%) | ILS(%) | p |
| 11 | 53 | 64 | NS | 22 | 113 | 131 | NS |
| 11 | 40 | 43 | NS | 22 | 100 | 138 | NS |
| 11 | 35 | 36 | NS | 20 | 88 | 219 | <0.0 1 |

Calculated values of LD₁₀ for etoposide alone (33 mg/kg) and for etoposide in combination with ICRF-187 (120 mg/kg) were used for treatment studies. 1×10⁵ L1210 tumour cells or 15×10⁶ Ehrlich's ascites tumour cells were inoculated i.p. on day 0 and mice were treated i.p. on day 4. The Mann Whitney test was used for statistical evaluation.

**Table 5A.**

| Calculations on the results from the treatment on mice bearing Ehrlich's tumour cells as shown in Table 5. | | | | | | | |
|---|---|---|---|---|---|---|---|
| **Ehrlich's tumour cells** | | | | **Ehrlich's tumour cells increased life span** | | | |
| Etoposide | | Etoposide + ICRF-187 | | Etoposide | | Etoposide + ICRF-187 | |
| Dose(mg/kg) | | Dose(mg/kg) | | Dose(mg/kg) | | Dose(mg/kg) | |
| 33 | | 120 + 125 | | 33 | | 120 + 125 | |
| N | ILS(%) | ILS(%) | p | | ILS(%) | increase in ILS(%) | ILS (%) |
| 11 | 53 | 64 | NS | | 100 | 21 | 121 |
| 11 | 40 | 43 | NS | | 100 | 8 | 108 |
| 11 | 35 | 36 | NS | | 100 | 3 | 103 |

**Table 5B.**

| Calculations on the results from the treatment on mice bearing L 1210 tumour cells as shown in Table 5. | | | | | | | |
|---|---|---|---|---|---|---|---|
| **L 1210 tumour cells** | | | | **L 1210 tumour cells increased life span** | | | |
| Etoposide | | Etoposide + ICRF-187 | | Etoposide | | Etoposide + ICRF-187 | |
| Dose(mg/kg) | | Dose(mg/kg) | | Dose(mg/kg) | | Dose(mg/kg) | |
| 33 | | 120 + 125 | | 33 | | 120 + 125 | |
| N | ILS(%) | ILS(%) | p | | ILS(%) | increase in ILS (%) | ILS (%) |
| 11 | 113 | 131 | NS | | 100 | 16 | 116 |
| 11 | 100 | 138 | NS | | 100 | 38 | 138 |
| 11 | 88 | 219 | <0.0 1 | | 100 | 149 | 249 |

**Table 6.**

| Treatment of mice inoculated i.c. with 10⁴ L1210 tumour cells. | | | |
|---|---|---|---|
| **L1210 tumour cells inoculated into the cerebrum** | | | |
| Etoposide | | Etoposide + ICRF-187 | |
| Dose (mg/kg) | | Dose (mg/kg) | |
| 33 | | 120 + 125 | |
| N | ILS(%) | ILS(%) | p |
| 18 | 13 | 38 | <0.01 |
| 18 | 21 | 38 | <0.01 |
| 18 | 13 | 63 | <0.01 |

Groups of 18 mice were inoculated on day 0 and treated on day 2. The Mann-Whitney test was used for statistical evaluation.

**Table 6A.**

| Treatment of mice inoculated i.c. with 15x10⁵ Ehrlich's ascites tumour cells. | | | |
|---|---|---|---|
| **Ehrlich's ascites tumour cells inoculated into the cerebrum** | | | |
| Etoposide | | Etoposide + ICRF-187 | |
| Dose (mg/kg) | | Dose (mg/kg) | |
| 33 | | 120 + 125 | |
| N | ILS(%) | ILS(%) | p |
| 9 | 11 | 33 | NS |
| | | | |
| | | | |

Groups of 9 mice were inoculated on day O and treated on day 2. The Mann-Whitney test was used for statistical evaluation.

The results in Table 6A show an increased life span from 11% to 33% corresponding to 300% compared to the increased life span in mice treated with topoisomerase II poison alone in an equitoxic dose.

**Table 7.**

| **Weight at nadir ( median and range in grams )** | | |
|---|---|---|
| | | |
| Control | 21.4 [20.4-21.8] | |
| ICRF-187 125 mg/kg | 21.2 [19.7-22.2] | |
| Etoposide 90 mg/kg | 15.4 [15.2-21.4] | |
| ICRF-187 + Etoposide 125 mg/kg + 90 mg/kg | 19.9 [18.0-20.8] | |

Groups of 5 mice were treated with drugs as stated and weighed for 11 consecutive days. Etoposide alone caused a median 6 g decrease in body weight, whereas the combination of drugs only resulted in a weight loss of 1.5 g.

### CLINICAL TRIAL

The following clinical test is performed:

### 1. Purpose

1. To determine the maximum tolerable dose (MTD) of etoposide in combination with a constant dose of ICRF-187 given once a week.
2. To evaluate the toxicity/tolerability of the combination treatment.
3. To evaluate the response rate of this treatment in patients having a histologically verified lung cancer and brain metastases verified by CT scanning.

### 2. Treatment doses and duration

| Dose escalation scheme | | |
|---|---|---|
| Escalation number | ICRF-187 mg/m² | Etoposide mg/m² |
| 1 | 1500 | 500 |
| 2 | 1500 | 1000 |
| 3 | 1500 | 1200 |
| 4 | 1500 | 1500 |
| 5 | 1500 | 2000 |
| 6 | 1500 | 2500 |
| 7 | 1500 | 3000 |

These doses are given once a week during hospitalization until progressive disease or pronounced toxicity [grade IV common toxicity criteria (CTC)]. One treatment series is constituted by weekly treatments during four consecutive weeks. After one series, the effect on tumour formation is evaluated. Then the weekly treatments are continued and the response evaluated every four weeks. The treatment is carried out in a total of 3 series, i.e. 12 treatments. One patient is included at the first dose level; after this unexpected haematological and non-haematological side effects are awaited for one week. Two more patients are included at the same dose level. If, at this dose level, there is no grade II CTC toxicity, the dose is escalated to the next dose level. If one out of three patients shows grade II CTC toxicity, three more patients are included at the level in question. If two out of these six patients show grade II CTC toxicity, MTD has been reached and the dose is reduced to 2/3 of the used escalation step. At the MTD thus determined, patients are included so that there are 12 patients in total at the level in question. The treatment is postponed for at the most 3 weeks in the case of non-restoration. No dose escalation is to be made with respect to the individual patient. The ICRF-187 dose has been selected so as to obtain a ratio of 1:1 between the substances.

### 3. Analysis criteria

### 3.a. Response

The ideal thing is that all measurable lesions are measured at each evaluation. If multiple lesions are found, this may not be possible, and under such circumstances up to 8 representative lesions must be selected.

Measurable disease (measurable uni- or bidimensionally):
1. Complete response (CR): Disappearance of all known disease, determined by two observations at intervals of at least 4 weeks.
2. Partial response (PR): In case of bidimensionally measurable disease, a reduction of at least 50% of the sum of the product of the largest perpendicular diameters of all measurable lesions, determined by two observations at intervals of at least 4 weeks. In case of unidimensionally measurable disease, a reduction of at least 50% of the sum of the largest diameters of all lesions, determined by two observations at intervals of at least 4 weeks.
3. No-change disease (NC): In case of bidimensionally measurable disease, a <50% reduction and a <25% increase of the sum of the product of the largest perpendicular diameters of all measurable lesions. In case of unidimensionally measurable disease, a <50% reduction and a <25% increase of the sum of the diameter of all lesions. No new lesions must arise.
4. Progressive disease (PD): A >25% increase of the size of at least one bidimensionally or unidimensionally measurable lesion or appearance of a new lesion.

### REFERENCES

2. Bork E, Ersbøll J, Dombernowsky P, Bergman B, Hansen M, Hansen HH (1991) Teniposide and etoposide in previously untreated small-cell lung cancer: A randomized study. J Clin Oncol, 9: 1627.
4. Cornfield J, Mantel N (1950) Some new aspects of the application of maximum likelihood to the calculation of the dosage response curve. Amer Statis Ass J. p. 181.
5. Danø K (1971) Development of resistance to daunomycin (NSC-82151) in Ehrlich's ascites tumour. Cancer Chemother Rep. 55: 133.
6. Donelli MG, Zucchetti M, D'Incalci M (1992) Do anticancer agents reach the tumour target in the human brain? Cancer Chemother Pharmacol. 30: 251.
7. Finlay GF, Wilson WR, Baguley BC (1989) Chemoprotection by 9-aminoacridine derivatives against the cytotoxicity of topoisomerase II-directed drugs. Eur J Cancer Clin Oncol. 25: 1695.
12. Herman EH, Witiak DT, Hellmann K, Waravdekar VS (1982) Biological properties of ICRF-159 and related bis(dioxopiperazine) compounds. Adv Pharmacol Chemother 19: 249.
13. Holm B, Jensen PB, Sehested M, Hansen HH (1994) In vivo inhibition of etoposide (VP-16) mediated apoptosis, toxicity, and antitumour effect by the topoisomerase II uncoupling anthracycline aclarubicin. Cancer Chemother Pharmacol. 34: 503.
14. Jensen PB, Roed H, Skovsgaard T, Friche E, Vindeløv LL, Hansen HH, Spang-Thomsen M (1990) Antitumour activity of the two epipodophyllotoxin derivatives VP-16 and VM-26 in preclinical systems: A comparison of in vitro and in vivo drug evaluation. Cancer Chemther Pharmacol. 27: 194.
15. Jensen PB, Sørensen BS, Demant EJF, Sehested M, Jensen PS, Vindeløv L, Hansen HH (1991) Antagonistic effect of aclarubicin on the cytotoxicity of etoposide and 4'-(9-acridinylamino)methanesulfon-m-anisidide in human small cell lung cancer cell lines and on topoisomerase II-mediated DNA cleavage. Cancer Res. 51: 3311.
16. Jensen PB, Jensen PS, Demant EJF, Friche E, Sørensen BS, Sehested M, Wassermann K, Vindeløv L, Westergaard O, Hansen HH (1991) Antagonistic effect of aclarubicin on daunorubicin-induced cytotoxicity in human small cell lung cancer cells: Relationship to DNA integrity and topoisomerase II. Cancer Res. 51: 5093.
17. Jensen PB, Sørensen BS, Sehested M, Demant EJF, Kjeldsen E, Friche E, Hansen HH (1993) Different modes of anthracycline interaction with topoisomerase II. Separate structures critical for DNA-cleavage, and for overcoming topoisomerase II-related drug resistance. Biochem Pharmacol. 45: 2025.
18. Jensen PB, Sørensen BS, Sehested M, Grue P, Demant EJF, Hansen HH (1994) Targeting the cytotoxicity of topoisomerase II-directed epipodophyllotoxins to tumour cells in acidic environments. Cancer Res. 54: 2959.
19. Liu LF (1989) DNA topoisomerase poisons as antitumour drugs. Annu Rev Biochem. 58: 351.
20. Pommier Y, Covey J, Kerrigan D, Mattes W, Markovits J, Kohn KW (1987) Role of DNA intercalation in the inhibition of purified mouse leukemia (L1210) DNA topoisomerase II by 9-aminoacridines. Biochem Pharmacol. 36: 3477.
21. Pommier Y, Kohn K (1989) Topoisomerase II inhibition by antitumour intercalators and demethylepipodophyllotoxins. In: R I Glazer (ed) Development in Cancer Chemotherapy. Boca Raton FL: CRC Press Inc. p 175.
22. Roca J, Ishida R, Berger JM, Andoh T, Wang JC (1994) Antitumour bis-dioxypiperazines inhibit yeast DNA topoisomerase II by trapping the enzyme in the form of a closed protein clamp. Proc Natl Acad Sci USA, 91: 1781.
23. Roca J, Wang JC (1994) DNA transport by a type II DNA topoisomerase: Evidence in favor of a two-gate mechanism. Cell, 77: 609.
24. Rowe T, Kupfer G, Ross W (1985) Inhibition of epipodophyllotoxin cytotoxicity by interference with topoisomerase-mediated DNA-cleavage. Biochem Pharmacol. 34: 2483.
25. Sehested M, Jensen PB, Sørensen BS, Holm B, Friche E, Demant EJF (1993) Antagonistic effect of the cardioprotector (+) -1,2-bis(3,5-dioxopiperazinyl-l-yl)propane (ICRF-187) on DNA breaks and cytotoxicity induced by the topoisomerase II directed drugs daunorubicin and etoposide (VP-16). Biochem Pharmacol. 46: 389.
26. Skipper HE, Schabel Jr FM, Wilcox WS (1964) Experimental evaluation of potential anticancer agents. XIII. On the criteria and kinetics associated with "curability" of experimental leukemia. Cancer Chemother Rep. 35: 1.
27. Tanabe K, Ikegami Y, Ishida R, Andoh T (1991) Inhibition of topoisomerase II by antitumour agents bis(2,6-dioxopiperazine) derivatives. Cancer Res. 51: 4903.
28. Tewey KM, Rowe TC, Yang L, Halligan BD, Liu LF (1984) Adriamycin-induced DNA damage mediated by mammalian DNA topoisomerase II. Science 226: 466.
29. Von Hoff DD, Soares N, Gormley P, Poplack DG (1980) Pharmacokinetics of ICRF-187 in the cerebrospinal fluid of subhuman primates. Cancer Treat Rep. 64: 734.

## Claims

1. Use of a combination of an effective tumour- or metastasis-killing amount of a topoisomerase II poison and a bis-dioxypiperazine for the preparation of medicaments for selectively killing tumour or metastatic cells within a compartment of a large mammal, wherein the topoisomerase II poison is not doxorubicin.

2. Use of a combination of an effective tumour- or metastasis-killing amount of a topoisomerase II polson and a bis-dioxypiperazine, or compositions comprising either entity for the preparation of medicaments for selectively killing tumour or metastatic cells within a compartment of a large mammal, wherein the topoisomerase II poison is not doxorubicin.

3. Use according to any one of the preceding claims, wherein the tumours or metastatic cells are within the central nervous system (CNS) of the large mammal.

4. Use according to claim 3, wherein the topoisomerase II poison and the bis-dioxypiperazine compound being selected and to be administered, with respect to their amounts, expressed in mg per m² of a human and with respect to the ratio between their amounts, in such a manner that in mice, the corresponding amounts (the conversion factor being so that 1 mg/kg in a human of a height of 180 cm and weighing 70 kg corresponding to a surface area of about 1.87 m², corresponds to 3 mg/kg in a mouse) will conform to the following criteria:
i) in mice inoculated into the cerebrum with 15×10⁴ Ehrlich's ascites tumour cells or 10×10⁴ L1210 leukaemia cells in a volume of 30 µl of isotonic sodium chloride inoculated on day 0 into the temporal region, treatment l.p. on day 2 with the amount of the topoisomerase II poison and with simultaneous treatment with the amount of the bis-dioxypiperazine compound results in an increased life span of at least 125% compared to the increased life span in mice treated with topoisomerase II poison alone in an equitoxic dose (the equitoxic dose being calculated as the dose which in healthy mice results in a similar lethality as the combination); and/or
ii) in mice treated with the topoisomerase II poison alone and combined with the bis-dioxypiperazine compound, respectively, the LD₁₀ of the combination is at least 25% higher than the LD₁₀ of the topoisomerase II poison alone.

5. Use according to any one of the preceding claims, wherein the tumour or metastatic cells are within the intraperitoneal cavity or intrapleural cavity of a human, wherein the topoisomerase II poison is not doxorubicin and daunorubicin.

6. Use according to claim 5, wherein the medicaments are to be administered in such a way that the topoisomerase II poison is administered locally to the intraperitoneal cavity or intrapleural cavity while the bis-dioxypiperazine compound is being administered systemically.

7. Use according to any of one of the preceding claims, wherein the topoisomerase II poison and the bis-dioxypiperazine compound are selected and to be administered to further conform to the following criterion:
iii) in mice inoculated i.p. with 15×10⁶ Ehrlich's ascites tumour cells on day 0, treatment i.p. on day 4 with the amount of the topoisomerase II poison and with simultaneous treatment with the amount of the bis-dioxypiperazine compound results in an increased life span of at the most 200%, such as at the most 150%, preferably at the most 125%, compared to the increased life span in mice treated with the topoisomerase II poison alone in an equitoxic dose as defined in claim 4.

8. Use according to any of one of the preceding claims, wherein the topoisomerase II poison is in a dosage amount equivalent to at least 200 mg/m² of etoposide, such as at least 500 mg/m² of etoposide, preferably at least 1000 mg/m² of etoposide, more preferably at least 1200 mg/m² of etoposide, even more preferred at least 1500 mg/m² of etoposide.

9. Use according to any one of the preceding claims, wherein the topoisomerase II poison is in a dosage amount equivalent to at least 2000 mg/m² of etoposide, such as at least 2500 mg/m² of etoposide, preferably at least 3000 mg/m² of etoposide, such as 3500 mg/m² of etoposide.

10. Use according to any one of the preceding claims, wherein the bis-dioxypiperazine compound is in a dosage amount equivalent to at least 200 mg/m² of ICRF-187 such as at least 500 mg/m² of ICRF-187, preferably at least 1000 mg/m² of ICRF-187, more preferably at least 1200 mg/m² of ICRF-187, even more preferred at least 1500 mg/m² of ICRF-187.

11. Use according to any one of the preceding claims, wherein the bis-dioxypiperazine compound is in a dosage amount equivalent of at least 2000 mg/m² of ICRF-187, such as at least 2500 mg/m² of ICRF-187, preferably at least 3000 mg/m² of ICRF-187, such as 3500 mg/m² of ICRF-187.

12. Use according to any one of the preceding claims, wherein the ratio between the dosage amount of topoisomerase II poison and the dosage amount of the bis-dioxypiperazine to be administered is in the range of 1:6 - 6:1, such as 1:4 - 4:1, preferably in the range of 1:3 - 3:1, calculated on the weight of the active drugs.

13. Use according to any one of the preceding claims, wherein the ratio between the dosage amount of topoisomerase II poison and the dosage amount of the bis-dioxypiperazine to be administered is in the range of 1:2 - 2:1, preferably in the range of 1.5:2 - 2:1.5, the most preferred ratio being 1:1, calculated on the weight of the active drugs.

14. Use according to any one of the preceding claims, wherein the topoisomerase II poison is to be administered in a high dosage amount such as a dosage of between 3 and 15 g/m².

15. Use according to any one of the preceding claims, wherein a human further receives a second therapy for stimulation of the hemopoietic tissue and/or receives transplantation of hemopoietic tissue.

16. Use according to any one of the preceding claims, wherein the topoisomerase II poison and the bis-dioxypiperazine are administered substantially simultaneously.

17. Use according to any of the preceding claims, wherein the bis-dioxypiperazine is administered prior to the administration of the topoisomerase II.

18. Use according to claim 17, wherein the bis-dioxypiperazine is administered in the period of from 72 hours before the administration of the topoisomerase II poison to 24 hours after, such as from 48 hours before to 12 hours after, more preferred from 24 hours before to 6 hours after, even more preferred from 12 hours before to 3 hours after, still more preferred from 6 hours before to 1 hour after, most preferred from 3 hours before to 1 hour after the administration of the topoisomerase II poison.

19. Use according to any one of the preceding claims, wherein the topoisomerase II poison and the bis-dioxypiperazine compound are selected and administered to conform to the following criterion:
i) In mice inoculated into the cerebrum with 15×10⁴ Ehrlich's ascites tumour cells in a volume of 30 µl of isotonic sodium chloride inoculated on day 0 into the temporal region, treatment l.p. on day 2 with the amount of the topolsomerase II poison and with simultaneous treatment with the amount of the bis-dioxypiperazine compound results in an increased life span of at least 150%, such as at least 200%, preferably at least 250%, such as about 300%, compared to the increased life span in mice treated with topoisomerase II poison alone in an equitoxic dose as defined in claim 4.

20. Use according to any one of the preceding claims, wherein the topoisomerase II poison and the bis-dioxypiperazine compound are selected and to be administered to conform to the following criterion:
ii) in mice treated with the topoisomerase II poison alone and combined with the bis-dioxypiperazine compound, respectively, the LD₁₀ of the combination is at least 50% higher, such as at least 100% higher, preferably at least 150% higher, than the LD₁₀ of the topoisomerase II poison alone.

21. Use according any of the preceding claims, wherein the topoisomerase II poison and the bis-dioxypiperazine compound are selected and to be administered to conform to the following criterion:
iia) in mice treated with the topolsomerase II poison alone and combined with the bis-dioxypiperazine compound, respectively, the LD₁₀ of the combination is at least 200% higher, even more preferred at least 250% higher, preferably at least 300% higher, more preferred at least 350% higher, such as 360% higher, than the LD₁₀ of the topoisomerase II poison alone.

22. Use according to any one of the preceding claims, wherein the topoisomerase II poison is administered in a schedule where an administration is separated from the next administration by at least 2 days (48 hours), such as by 3 days.

23. Use according to any one of the preceding claims, wherein the topolsomerase II poison is administered in a schedule where an administration is separated from the next administration by at least 4 days, such as by 5 days.

24. Use according to any one of the preceding claims, wherein the topoisomerase II poison is administered in a schedule where each administration is separated from the next administration by at least 12 hours, such as about 24 hours or 2 days.

25. Use according to claim 24, wherein each administration is separated from the next administration by from about 3 days to about 3 weeks.

26. Use according to claim 25, wherein each administration is separated from the next administration by about 7 days.

27. Use according to any one of the preceding claims, wherein the topoisomerase II poison is etoposide.

28. Use according to any one of the preceding claims, wherein the topoisomerase II poison is teniposide (VM-26).

29. Use according to any one of the preceding claims, wherein the topoisomerase II poison is etoposide-phosphate.

30. Use according to any one of the preceding claims, wherein the topoisomerase II poison is daunorubicin.

31. Use according to any one of the preceding claims, wherein the bis-dioxypiperazine is ICRF-187.

32. Use according to any one of the preceding claims, wherein the topoisomerase II poison is selected from the group consisting of etoposide (VP-16), etoposide-phosphate, teniposide (VM-26), m-AMSA, daunorubicin, and mitoxantrone.

33. Use according to any one of the preceding claims, wherein a human further receives a topoisomerase II poison alone and/or in combination with one or more other anticancer agents.

34. A pharmaceutical kit for selectively killing tumour or metastatic cells within the central nervous system of a human, said kit consisting of a single infusion system comprising
a) a dosage unit of a bis-dioxypiperazine and a pharmaceutically acceptable carrier, and
b) a dosage unit of topoisomerase II poisons except doxorubicin and a pharmaceutically acceptable carrier
wherein the two dosage units are separated in two individual bags.

## Patentansprüche

1. Verwendung einer Kombination einer wirksamen Tumor- oder Metastasen abtötenden Menge eines Topoisomerase II Giftes sowie eines Bis-Dioxypiperazins, für die Herstellung von Medikamenten zur selektiven Abtötung von Tumor- oder metastasenbildenden Zellen innerhalb eines Kompartiments eines großen Säugetiers inklusive Mensch, wobei das Topoisomerase II Gift nicht Doxorubicin ist.

2. Verwendung einer Kombination einer wirksamen Tumor- oder Metastasen abtötenden Menge eines Topoisomerase II Giftes sowie eines Bis-Dioxypiperazins, oder von beides umfassenden Zusammensetzungen, für die Herstellung von Medikamenten zur selektiven Abtötung von Tumor- oder metastasenbildenden Zellen innerhalb eines Kompartiments eines großen Säugetiers inklusive Mensch, wobei das Topoisomerase II Gift nicht Doxorubicin ist.

3. Verwendung nach einem der vorhergehenden Ansprüche, wobei die Tumoren oder metastasenbildenden Zellen innerhalb des Zentralnervensystems (ZNS) des großen Säugetiers vorliegen.

4. Verwendung nach Anspruch 3, wobei das Topoisomerase II Gift und die Bis-Dioxypiperazin-Verbindung ausgewählt sind und bezüglich ihrer Mengen, ausgedrückt in mg pro m² eines Menschen und bezüglich des Verhältnisses zwischen ihren Mengen, auf solche Weise verabreicht werden, dass bei Mäusen (wobei der Umwandlungsfaktor so ist, dass 1 mg pro kg beim Menschen einer Größe von 1,80 m und mit einem Gewicht von 70 kg, entsprechend einer Oberfläche von etwa 1,87 m², 3 mg pro kg bei der Maus entsprechen) die entsprechenden Mengen den folgenden Kriterien entsprechen:
i) bei Mäusen inokuliert ins Zerebrum mit 15 x 10⁴ Ehrlich-Ascites Tumorzellen oder 10 x 10⁴ L1210 Leukämiezellen in einem Volumen von 30 µl isotonischer Natriumchloridlösung, inokuliert am Tage 0 in den temporalen Bereich, Behandlung i. p. am Tag 2 mit der Menge des Topoisomerase II Giftes und bei gleichzeitiger Behandlung mit der Menge der Bis-Dioxypiperazin-Verbindung resultiert eine erhöhte Lebensspanne von mindestens 125 %, im Vergleich zur erhöhten Lebensspanne von Mäusen, die mit Topoisomerase II Gift allein in einer äquitoxischen Dosis behandelt wurden (wobei die äquitoxische Dosis berechnet wird als die Dosis, die bei gesunden Mäusen zu einer ähnlichen Letalität wie die Kombination führt); und/oder
ii) bei Mäusen, die mit dem Topoisomerase II Gift allein bzw. kombiniert mit der Bis-Dioxypiperazin-Verbindung behandelt wurden, ist der LD₁₀ der Kombination mindestens 25 % größer als der LD₁₀ des Topoisomerase II Giftes allein.

5. Verwendung gemäß einem der vorhergehenden Ansprüche, wobei die Tumoroder metastasenbildenden Zellen innerhalb des intraperitonealen Hohlraums oder des intrapleuralen Hohlraums eines Menschen sind, und wobei das Topoisomerase II Gift nicht Doxorubicin und Daunorubicin ist.

6. Verwendung nach Anspruch 5, wobei die Medikamente auf solche Weise verabreicht werden, dass das Topoisomerase II Gift lokal am intraperitonealen Hohlraum oder am intrapleuralen Hohlraum verabreicht wird, während die Bis-Dioxypiperazin-Verbindung systemisch verabreicht wird.

7. Verwendung nach einem der vorhergehenden Ansprüche, wobei das Topoisomerase II Gift und die Bis-Dioxypiperazin-Verbindung ausgewählt sind und verabreicht werden um ferner dem folgenden Kriterium zu entsprechen:
iii) bei Mäusen inokuliert i. p. mit 15 x 10⁶ Ehrlich-Ascites Tumorzellen am Tag 0, Behandlung i. p. am Tag 4 mit der Menge an Topoisomerase II Gift und mit gleichzeitiger Behandlung mit der Menge der Bis-Dioxypiperazin-Verbindung, führt die Behandlung zu einer erhöhten Lebensspanne von höchstens 200 %, wie etwa höchstens 150 %, vorzugsweise höchstens 125 %, verglichen mit der erhöhten Lebensspanne von Mäusen, die mit dem Topoisomerase II Gift allein in einer äquitoxischen Dosis, wie in Anspruch 4 definiert, behandelt wurden.

8. Verwendung nach einem der vorhergehenden Ansprüche, wobei das Topoisomerase II Gift in einer Dosismenge vorliegt, die äquivalent ist zu mindestens 200 mg pro m² Etoposid, wie etwa mindestens 500 mg pro m² Etoposid, vorzugsweise mindestens 1000 mg pro m² Etoposid, stärker bevorzugt mindestens 1200 mg pro m² Etoposid, und insbesondere bevorzugt mindestens 1500 mg pro m² Etoposid.

9. Verwendung nach einem der vorhergehenden Ansprüche, wobei das Topoisomerase II Gift in einer Dosismenge vorliegt, die äquivalent ist zu mindestens 2000 mg pro m² Etoposid ist, wie etwa mindestens 2500 mg pro m² Etoposid, vorzugsweise mindestens 3000 mg pro m² Etoposid, und insbesondere etwa 3500 mg pro m² Etoposid.

10. Verwendung nach einem der vorhergehenden Ansprüche, wobei die Bis-Dioxypiperazin-Verbindung in einer Dosismenge vorliegt, die äquivalent ist zu mindestens 200 mg pro m² ICRF-187, wie etwa mindestens 500 mg pro m² ICRF-187, vorzugsweise mindestens 1000 mg pro m² ICRF-187, weiter bevorzugt mindestens 1200 mg pro m² ICRF-187, und insbesondere bevorzugt mindestens 1500 mg pro m² ICRF-187.

11. Verwendung nach einem der vorhergehenden Ansprüche, wobei die Bis-Dioxypiperazin-Verbindung in einer Dosismenge vorliegt, die äquivalent ist zu mindestens 2000 mg pro m² ICRF-187, wie etwa mindestens 2500 mg pro m² ICRF-187, vorzugsweise mindestens 3000 mg pro m² ICRF-187, insbesondere bevorzugt etwa 3500 mg pro m² ICRF-187.

12. Verwendung nach einem der vorhergehenden Ansprüche, wobei das Verhältnis zwischen der Dosismenge des Topoisomerase II Giftes und der Dosismenge des Bis-Dioxypiperazins, die verabreicht werden sollen, im Bereich von 1:6-6:1, wie etwa 1:4 bis 4:1, vorzugsweise im Bereich von 1:3 - 3:1 liegt, berechnet nach dem Gewicht der aktiven Wirkstoffe.

13. Verwendung nach einem der vorhergehenden Ansprüche, wobei das Verhältnis zwischen der Dosismenge des Topoisomerase II Giftes und der Dosismenge des Bis-Dioxypiperazins, die verabreicht werden sollen, im Bereich von 1:2 - 2:1, vorzugsweise im Bereich von 1,5:2-2:1,5, besonders bevorzugt bei 1:1 liegt, berechnet nach dem Gewicht der aktiven Wirkstoffe.

14. Verwendung nach einem der vorhergehenden Ansprüche, wobei das zu verabreichende Topoisomerase II Gift in einer hohen Dosismenge vorliegt, wie etwa einer Dosierung von zwischen 3 und 15 g pro m².

15. Verwendung nach einem der vorhergehenden Ansprüche, wobei ein Mensch ferner eine Zweittherapie zur Stimulierung des hämopoietischen Gewebes bekommt und/oder eine Transplantation des hämopoietischen Gewebes empfängt.

16. Verwendung nach einem der vorhergehenden Ansprüche, wobei das Topoisomerase II Gift und das Bis-Dioxypiperazin im wesentlichen gleichzeitig verabreicht werden.

17. Verwendung nach einem der vorhergehenden Ansprüche, wobei das Bis-Dioxypiperazin vor der Verabreichung der Topoisomerase II verabreicht wird.

18. Verwendung nach Anspruch 17, wobei das Bis-Dioxypiperazin im Zeitraum von 72 Stunden vor der Verabreichung des Topoisomerase II Giftes bis 24 Stunden danach, wie etwa von 48 Stunden vor bis 12 Stunden danach, stärker bevorzugt von 24 Stunden davor bis 6 Stunden danach, weiters bevorzugt von 12 Stunden davor bis 3 Stunden danach, noch weiter bevorzugt von 6 Stunden davor bis 1 Stunde danach, insbesondere bevorzugt von 3 Stunden davor bis 1 Stunde nach der Verabreichung des Topoisomerase II Giftes verabreicht wird.

19. Verwendung nach einem der vorhergehenden Ansprüche, wobei das Topoisomerase II Gift und die Bis-Dioxypiperazin-Verbindung ausgewählt sind und verabreicht werden um dem folgenden Kriterium zu entsprechen:
i) bei Mäusen inokuliert in das Zerebrum mit 15 x 10⁴ Ehrlich-Ascites Tumorzellen in einem Volumen von 30 µl isotonischer Natriumchloridlösung, inokuliert am Tag 0 in den temporalen Bereich, Behandlung i. p. am Tag 2 mit der Menge an Topoisomerase II Gift und bei gleichzeitiger Behandlung mit der Menge der Bis-Dioxypiperazin-Verbindung, resultiert die Behandlung in einer erhöhten Lebensspanne von mindestens 150 %, wie etwa mindestens 200 %, vorzugsweise mindestens 250 %, wie etwa 300 %, im Vergleich zur erhöhten Lebensspanne bei Mäusen, die mit Topoisomerase II Gift allein in einer äquitoxischen Dosis, wie in Anspruch 4 definiert, behandelt wurden.

20. Verwendung nach einem der vorhergehenden Ansprüche, wobei das Topoisomerase II Gift und die Bis-Dioxypiperazin-Verbindung ausgewählt sind und verabreicht werden um den folgenden Kriterium zu entsprechen:
ii) bei Mäusen behandelt mit dem Topoisomerase II Gift allein bzw. kombiniert mit der Bis-Dioxypiperazin-Verbindung, ist der LD₁₀ der Kombination mindestens 50 % höher, wie etwa mindestens 100 % höher, vorzugsweise mindestens 150 % höher, als der LD₁₀ des Topoisomerase II Giftes allein.

21. Verwendung nach einem der vorhergehenden Ansprüche, wobei das Topoisomerase II Gift und die Bis-Dioxypiperazin-Verbindung ausgewählt sind und verabreicht werden um dem folgenden Kriterium zu entsprechen:
iia) bei Mäusen behandelt mit dem Topoisomerase II Gift allein bzw. kombiniert mit der Bis-Dioxypiperazin-Verbindung, ist der LD₁₀ der Kombination um mindestens 200 % höher, stärker bevorzugt mindestens 250 % höher, vorzugsweise mindestens 300 % höher, weiters bevorzugt mindestens 350 %. höher, wie etwa 360 % höher, als der LD₁₀ des Topoisomerase II Giftes allein.

22. Verwendung nach einem der vorhergehenden Ansprüche, wobei das Topoisomerase II Gift gemäß einem Zeitplan verabreicht wird, worin eine Verabreichung von der nächsten Verabreichung um mindestens 2 Tage (48 Stunden), wie etwa um 3 Tage, getrennt liegt.

23. Verwendung nach einem der vorhergehenden Ansprüche, wobei das Topoisomerase II Gift gemäß einem Zeitplan verabreicht wird, in dem eine Verabreichung von der nächsten Verabreichung um mindestens 4 Tage, wie etwa um 5 Tage, getrennt liegt.

24. Verwendung nach einem der vorhergehenden Ansprüche, wobei das Topoisomerase II Gift gemäß einem Zeitplan verabreicht wird, in dem jede Verabreichung von der nächsten Verabreichung um mindestens 12 Stunden, wie etwa 24 Stunden oder 2 Tage, getrennt ist.

25. Verwendung nach Anspruch 24, wobei jede Verabreichung von der nächsten Verabreichung um etwa 3 Tage bis etwa 3 Wochen getrennt ist.

26. Verwendung nach Anspruch 25, wobei jede Verabreichung von der nächsten Verabreichung um etwa 7 Tage getrennt liegt.

27. Verwendung nach einem der vorhergehenden Ansprüche, wobei das Topoisomerase II Gift Etoposid ist.

28. Verwendung nach einem der vorhergehenden Ansprüche, wobei das Topoisomerase II Gift Teniposid (VM-26) ist.

29. Verwendung nach einem der vorhergehenden Ansprüche, wobei das Topoisomerase II Gift Etoposid-Phosphat ist.

30. Verwendung nach einem der vorhergehenden Ansprüche, wobei das Topoisomerase II Gift Daunorubicin ist.

31. Verwendung nach einem der vorhergehenden Ansprüche, wobei das Bis-Dioxypiperazin ICRF-187 ist.

32. Verwendung nach einem der vorhergehenden Ansprüche, wobei das Topoisomerase II Gift aus der Gruppe bestehend aus Etoposid (VP-16), Etoposid-Phosphat, Teniposid (VM-26), m-AMSA, Daunorubicin sowie Mitoxantron ausgewählt ist.

33. Verwendung nach einem der vorhergehenden Ansprüche, wobei ein Mensch ferner ein Topoisomerase II Gift allein und/oder in Kombination mit einem oder mehreren anderen Anti-Krebsmitteln erhält.

34. Pharmazeutischer-Kit zur selektiven Abtötung von Tumor- oder metastasenbildenden Zellen im zentralen Nervensystem eines Menschen, wobei der Kit aus einem einfachen Infusionssystem besteht, welches umfasst:
a) eine Dosiseinheit eines Bis-Dioxypiperazins sowie einen pharmazeutisch akzeptablen Träger, und
b) eine Dosiseinheit eines Topoisomerase II Giftes mit Ausnahme von Doxorubicin sowie einen pharmazeutisch akzeptablen Träger,
wobei die beiden Dosiseinheiten in zwei einzelnen Verpackungen getrennt vorliegen.

## Revendications

1. Utilisation d'une quantité efficace pour détruire les tumeurs ou les métastases d'une association d'un poison topo-isomérase II et d'une bis-dioxypipérazine pour la préparation de médicaments destinés à détruire sélectivement les cellules tumorales ou métastatiques dans un compartiment d'un grand mammifère, dans laquelle le poison topo-isomérase II n'est pas la doxorubicine.

2. Utilisation d'une quantité efficace pour détruire les tumeurs ou les métastases d'une association d'un poison topo-isomérase II et d'une bis-dioxypipérazine, ou de compositions comprenant chacune des entités pour la préparation de médicaments destinés à détruire sélectivement les cellules tumorales ou métastatiques dans un compartiment d'un grand mammifère, dans laquelle le poison topo-isomérase II n'est pas la doxorubicine.

3. Utilisation selon l'une quelconque des revendications précédentes, dans laquelle les cellules tumorales ou métastatiques se trouvent dans le système nerveux central (SNC) d'un grand mammifère.

4. Utilisation selon la revendication 3, dans laquelle le poison topo-isomérase II et le composé bis-dioxypipérazine sont sélectionnés et doivent être administrés, en ce qui concerne leurs quantités, exprimées en mg par m² d'un homme, et en ce qui concerne le rapport entre leurs quantités, de telle sorte que chez la souris, les quantités correspondantes (le facteur de conversion étant tel que 1 mg/kg chez un homme mesurant 180 cm et pesant 70 kg correspondant à une surface d'environ 1,87 m², correspond à 3 mg/kg chez une souris) soient conformes aux critères suivants :
I) Chez des souris ayant reçu une inoculation dans le cerveau de 15 x 10⁴ cellules de tumeur ascitique d'Ehrlic ou de 10 x 10⁴ cellules de leucémie L1210 dans un volume de 30 µl de chlorure de sodium isotonique inoculées le jour 0 dans la région temporale, un traitement intra-péritonéal le jour 2 avec la quantité de poison topo-isomérase II et avec le traitement simultané par la quantité de composé bis-dioxypipérazine entraîne une augmentation de l'espérance de vie d'au moins 125 % par rapport à l'augmentation de l'espérance de vie chez des souris traitées par le poison topo-isomérase II seul à une posologie équitoxique (la posologie équitoxique étant calculée comme la posologie qui chez des souris saines entraîne une létalité similaire à l'association) ; et/ou
II) Chez des souris traitées par le poison topo-isomérase II seul et associé au composé bis-dioxypipérazine respectivement, la DL₁₀ de l'association est plus élevée d'au moins 25 % que la DL₁₀ du poison topo-isomérase II seul.

5. Utilisation selon l'une quelconque des revendications précédentes, dans laquelle les cellules tumorales ou métastatiques se trouvent dans la cavité intra-péritonéale ou la cavité intra-pleurale d'un homme,
dans laquelle le poison topo-isomérase II n'est ni la doxorubicine ni la daunorubicine.

6. Utilisation selon la revendication 5, dans laquelle les médicaments doivent être administrés de telle manière que le poison topo-isomérase II est administré localement dans la cavité intra-péritonéale ou dans la cavité intra-pleurale tandis que le composé bis-dioxypipérazine est administré par voie systémique.

7. Utilisation selon l'une quelconque des revendications précédentes, dans laquelle le poison topo-isomérase II et le composé bis-dioxypipérazine sont sélectionnés et doivent être administrés pour se conformer en outre au critère suivant :
III) Chez des souris ayant reçu une inoculation intra-péritonéale de 15 x 10⁵ cellules de tumeur ascitique d'Ehrlic le jour 0, un traitement intra-péritonéal le jour 4 avec la quantité de poison topo-isomérase II et avec un traitement simultané par la quantité de composé bis-dioxypipérazine entraîne une augmentation de l'espérance de vie au plus de 200 %, par exemple au plus de 150 %, de préférence au plus de 125 %, par rapport à l'augmentation de l'espérance de vie chez des souris traitées par le poison topo-isomérase II seul à une posologie équitoxique selon la définition de la revendication 4.

8. Utilisation selon l'une quelconque des revendications précédentes, dans laquelle le poison topo-isomérase II est à un dosage d'une quantité équivalente à au moins 200 mg/m² d'étoposide, par exemple au moins 500 mg/m² d'étoposide, de préférence au moins 1 000 mg/m² d'étoposide, plus préférablement au moins 1 200 mg/m² d'étoposide, encore plus préférablement au moins 1 500 mg/m² d'étoposide.

9. Utilisation selon l'une quelconque des revendications précédentes, dans laquelle le poison topo-isomérase II est à un dosage d'une quantité équivalente à au moins 2 000 mg/m² d'étoposide, par exemple au moins 2 500 mg/m² d'étoposide, de préférence au moins 3 000 mg/m² d'étoposide, par exemple 3 500 mg/m² d'étoposide.

10. Utilisation selon l'une quelconque des revendications précédentes, dans laquelle le composé bis-dioxypipérazine est à un dosage d'une quantité équivalente à au moins 200 mg/m² d'ICRF-187, par exemple au moins 500 mg/m² d'ICRF-187, de préférence au moins 1 000 mg/m² d'ICRF-187, plus préférablement au moins 1 200 mg/m² d'ICRF-187, encore plus préférablement au moins 1 500 mg/m² d'ICRF-187.

11. Utilisation selon l'une quelconque des revendications précédentes, dans laquelle le composé bis-dioxypipérazine est à un dosage d'une quantité équivalente à au moins 2 000 mg/m² d'ICRF-187, par exemple au moins 2 500 mg/m² d'ICRF-187, de préférence au moins 3 000 mg/m³ d'ICRF-187, par exemple 3 500 mg/m² d'ICRF-187.

12. , Utilisation selon l'une quelconque des revendications précédentes, dans laquelle le rapport entre la quantité de poison topo-isomérase II et la quantité de bis-dioxypipérazine devant être administrées est compris dans l'intervalle de 1:6 - 6:1, par exemple 1:4 - 4:1, de préférence dans l'intervalle de 1:3 - 3:1, calculé sur le poids des principes actifs.

13. Utilisation selon l'une quelconque des revendications précédentes, dans laquelle le rapport entre la quantité de poison topo-isomérase II et la quantité de bis-dioxypipérazine devant être administrées est compris dans l'intervalle de 1:2 - 2:1, de préférence dans l'intervalle de 1,5:2 - 2;1,5, le rapport le plus préférable étant 1:1, calculé sur le poids des principes actifs.

14. Utilisation selon l'une quelconque des revendications précédentes, dans laquelle le poison topo-isomérase II doit être administré à une posologie élevée par exemple une posologie comprise entre 3 et 15 g/m².

15. Utilisation selon l'une quelconque des revendications précédentes, dans laquelle un homme reçoit en outre un second traitement pour la stimulation du tissu hématopoïétique et/ou reçoit une transplantation de tissu hématopoiétique.

16. Utilisation selon l'une quelconque des revendications précédentes, dans laquelle le poison topo-isomérase II et la bis-dioxypipérazine sont administrés pratiquement de manière simultanée.

17. Utilisation selon l'une quelconque des revendications précédentes, dans laquelle la bis-dioxypipérazine est administrée avant l'administration de la topo-isomérase II.

18. Utilisation selon la revendication 17, dans laquelle la bis-dioxypipérazine est administrée dans une période de 72 heures avant l'administration du poison topo-isoméraee II jusqu'à 24 heures après, par exemple de 48 heures avant jusqu'à 12 heures après, plus préférablement de 24 heures avant jusqu'à 6 heures après, encore plus préférablement de 12 heures avant jusqu'à 3 heures après, toujours plus préférablement entre 6 heures avant jusqu'à une heure après, de manière préférée entre toutes de 3 heures avant jusqu'à 1 heure après l'administration du poison topo-isomérase II.

19. Utilisation selon l'une quelconque des revendications précédentes, dans laquelle le poison topo-isomérase II et le composé bis-dioxypipérazine sont sélectionnés et administrés pour se conformer au critère suivant :
i) Chez des souris ayant reçu une inoculation de 15 x 10⁴ cellules de tumeur ascitique d'Ehrlich dans un volume de 30 µl de chlorure de sodium isotonique inoculées le jour 0 dans la région temporal, un traitement intra-péritonéal le jour 2 avec la quantité de poison topo-isomérase II et avec un traitement simultané par la quantité de composé bis-dioxypipérazine entraîne une augmentation de l'espérance de vie d'au moins 150 %, par exemple au moins 200 %, de préférence au moins 250 %, par exemple environ 300 %, par rapport à l'augmentation de l'espérance de vie chez des souris traitées par le poison topo-isomérase II seul à une posologie équitoxique selon la définition de la revendication 4.

20. Utilisation selon l'une quelconque des revendications précédentes, dans laquelle le poison topo-isomérase II et le composé bis-dioxypipérazine sont sélectionnés et doivent être administrés pour se conformer au critère suivant :
ii) Chez des souris traitées par le poison topo-isomérase II seul et associé au composé bis-dioxypipérazine respectivement, la DL₁₀ de l'association est plus élevée d'au moins 50 %, par exemple plus élevée d'au moins 100 %, de préférence plus élevée d'au moins 150 %, que la DL₁₀ du poison topo-isomérase II seul.

21. Utilisation selon l'une quelconque des revendications précédentes, dans laquelle le poison topo-isomérase II et le composé bis-dioxypipérazine sont sélectionnés et doivent être administrés pour se conformer au critère suivant :
iia) Chez des souris traitées par le poison topo-isomérase II seul et associé au composé bis-dioxypipérazine respectivement, la DL₁₀ de l'association est plus élevée d'au moins 200 %, encore plus préférablement plus élevée d'au moins 250 %, de préférence plus élevée d'au moins 300 %, plus préférablement plus élevée d'au moins 350 %, par exemple plus élevée de 360 %, que la DL₁₀ du poison topo-isomérase II seul.

22. Utilisation selon l'une quelconque des revendications précédentes, dans laquelle le poison topo-isomérase II est administré selon un calendrier dans lequel une administration est séparée de l'administration suivante d'au moins 2 jours (48 heures), par exemple de 3 jours.

23. Utilisation selon l'une quelconque des revendications précédentes, dans laquelle le poison topo-isomérase II est administré selon un calendrier dans lequel une administration est séparée de l'administration suivante d'au moins 4 jours, par exemple de 5 jours.

24. Utilisation selon l'une quelconque des revendications précédentes, dans laquelle le poison topo-isomérase II est administré selon un calendrier dans lequel chaque administration est séparée de l'administration suivante d'au moins 12 heures, par exemple d'environ 24 heures ou de 2 jours.

25. Utilisation selon la revendication 24, dans laquelle chaque administration est séparée de l'administration suivante d'environ 3 jours à environ 3 semaines.

26. Utilisation selon la revendication 25, dans laquelle chaque administration est séparée de l'administration suivante d'environ 7 jours.

27. Utilisation selon l'une quelconque des revendications précédentes, dans laquelle le poison topo-isomérase II est l'étoposide.

28. Utilisation selon l'une quelconque des revendications précédentes, dans laquelle le poison topo-isomérase II est le téniposide (VM-26).

29. Utilisation selon l'une quelconque des revendications précédentes, dans laquelle le poison topo-isomérase II est l'étoposide phosphate.

30. Utilisation selon l'une quelconque des revendications précédentes, dans laquelle le poison topo-isomérase II est la daunorubicine.

31. Utilisation selon l'une quelconque des revendications précédentes, dans laquelle la bis-dioxypipérazine est l'ICRF-187.

32. Utilisation selon l'une quelconque des revendications précédentes, dans laquelle le poison topo-isomérase II est sélectionné dans le groupe comprenant l'étoposide (VP-16), l'étoposide phosphate, le téniposide (VM-26), l'amsacrine, la daunorubicine, et la mitoxantrone.

33. Utilisation selon l'une quelconque des revendications précédentes, dans laquelle un homme reçoit également un poison topo-isomérase II seul et/ou en association avec un ou plusieurs autres agents anticancéreux.

34. Kit pharmaceutique pour la destruction sélective des cellules tumorales ou métastatiques dans le système nerveux central d'un homme, ledit kit consistant en un système de perfusion unique comprenant
a) une unité d'administration d'une bis-dioxypipérazine et d'un porteur pharmaceutiquement acceptable, et
b) une unité d'administration de poisons topo-isomérase II, à l'exception de la doxorubicine, et un porteur pharmaceutiquement acceptable
dans lequel les deux unités d'administration sont séparées en deux poches individuelles.
